# EUROPEAN PATENT APPLICATION

(11) **EP 1 754 694 A1**
(43) Date of publication of application: **21.02.2007**
(21) Application number: 05017339.2
(22) Date of filing: 10.08.2005
(51) Int. Cl.: C07C 2/08

(54) **Method for deactivation and removal of catalytic components in the oligomerisation of ethylene**

(71) Applicant: Linde AG, 65189 Wiesbaden (DE); Saudi Basic Industries Corporation, Riyadh 11422 (SA)
(72) Inventor: Fritz, Peter, Dr., 82008 Unterchaching (DE); Bölt, Heinz, 82515 Wolfratshausen (DE); Wellenhofer, Anton, 81247 München (DE); Hackner, Holger, 80807 München (DE); Thiede, Heiko, 81669 München (DE); Mosa, Fuad, 11422 Riyadh (SA); Ali, Talal, Dr., 11422 Riyadh (SA); Al-Otaibi, Sultan, 11422 Riyadh (SA)
(74) Representative: Winkler, Andreas Fritz Ernst

(57) **Abstract**

The present invention relates to a method for deactivation and removal of catalytic components in the oligomerisation of ethylene to form linear alpha-olefins, comprising the steps of:
a) obtaining an organic phase outlet stream from an oligomerisation reactor comprising organic solvent, catalyst, dissolved ethylene and linear alpha-olefins;
b) mixing the outlet stream obtained in step a) with an aqueous phase basic solution to deactivate the catalyst compontents; and
c) separating the linear alpha-olefins containing organic phase from the aqueous basic phase, the aqueous basic phase containing the deactivated catalyst components.

## Description

The present invention relates to a method for deactivation and removal of catalytic components in the oligomerisation of ethylene to form linear alpha-olefins (LAO).

Methods for the preparation of linear alpha-olefins by oligomerizing of ethylene in the presence of organic solvents and homogeneous catalysts are widely known in the art. For example, DE 43 38 414 C1 discloses a respective method utilizing an empty tubular reactor wherein ethylene is introduced into the bottom of the reactor, the products of the oligomerisation being discharged from the lower part of the reactor together with solvent, dissolved ethylene and catalyst.

A common catalyst which may be utilized in the oligomerisation of ethylene is a two component catalyst comprising a zirconium compound, such as zirconium tetraisobutyrate, and an aluminum compound as activator, such as ethyl aluminum sesquichloride, as disclosed, e.g., in DE 43 38 416 C1 and DE 198 12 066 A1.

As already discussed in the prior art, it is a major problem of the oligomerisation method to rapidly deactivate the catalyst after having left the oligomerisation reactor and its removal from the desired product stream of linear alpha-olefins. In DE 43 38 414 C1 the deactivation of the catalyst by addition of water, alcohol or fatty acids is disclosed. However, the separation of the deactivated catalyst components from the organic phase containing linear alpha-olefins is still complicated and cost-intensive.

DE 198 07 226 A1 discloses a process for deactivation of complex metal organic catalysts in homogeneous processes, such as the oligomerization of ethylene, wherein the obtained product solution is mixed with metal hydroxide in a protonic solvent, wherein the activation and isolation of the catalysts from the organic phase are carried out in one step. In this process, the amount of the aqueous phase is kept relatively low, just enough to ensure the deactivation of the catalyst. This small amount of aqueous phase could be dissolved or entrained in the organic phase, but is too small for the formation of a separate phase.

It is therefore an object of the present invention to provide a method for deactivation and removal of catalytic components in the oligomerisation of ethylene which overcomes the disadvantages of the prior art. Especially, a method shall be provided wherein the deactivated catalyst components may be easily and cost-effectively removed from the product stream. Further, the deactivated and separated catalyst components shall be preferably re-usable as, e.g., fine chemicals.

This object is achieved by a method for deactivation and removal of catalytic components in the oligomerisation of ethylene to form linear alpha-olefins, comprising the steps of:
a) obtaining an organic phase outlet stream from an oligomerisation reactor comprising organic solvent, catalyst, dissolved ethylene and linear alpha-olefins;
b) mixing the outlet stream obtained in step a) with an aqueous phase basic solution to deactivate the catalyst compontents; and
c) separating the linear alpha-olefins containing organic phase from the aqueous basic phase, the aqueous basic phase containing the deactivated catalyst components.

In a preferred embodiment mixing in step b) is carried out in a static mixer.

Preferably, separating in step c) is carried out in a gravity separator.

Moreover, the aqueous basic phase may contain alkali metal hydroxide, preferably NaOH and/or KOH, NH₃, organic amines or mixtures thereof.

In a further embodiment the aqueous basic phase obtained in step c) is recycled into mixing step b).

Preferably deactivated solid catalytic components are separated from deactivated dissolved catalytic components.

In one aspect the solid catalytic components and/or the dissolved catalytic components obtained are dried, neutralized and/or precipitated.

In addition it is also preferred that the linear alpha-olefins containing organic phase obtained in step c) is washed with water.

Surprisingly, it was found that utilizing the inventive method the catalytic components may be easily deactivated and separated from the product stream containing linear alpha-olefins. As the linear alpha-olefins are dissolved in the organic solvent and the deactivated catalyst components are dissolved in the aqueous basic phase (or precipitated as ZrO₂), the deactivated catalyst components may be separated from the organic phase by gravity separation. Additionally, the deactivated catalyst components obtained may be preferably processed further and separated from each other to be re-usable as, e.g., fine chemicals. In the inventive method, larger quantities of the aqueous solvent are employed which allow a phase separation between organic and aqueous phases, since these large quantities of aqueous phase cannot be dissolved in the organic phase.

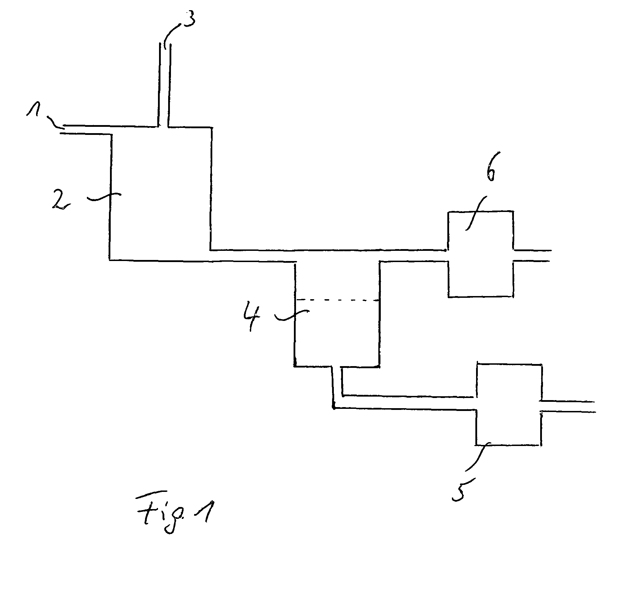

Additional features and advantages of the inventive method will now become apparent from the detailed description of a preferred embodiment thereof with reference to the accompanying drawing, wherein Figure 1 schematically illustrates an embodiment of the inventive method.

Figure 1 illustrates schematically an inventive method for deactivation and removal of catalytic components in the oligomerisation of ethylene. An outlet stream comprising organic solvent, catalyst, dissolved ethylene and linear alpha-olefins is introduced via line 1 into a static mixing vessel 2. As catalyst a well known Zr-compound and a Al-compound as activator may be used, e.g. zirconium tetraisobutyrate and ethyl aluminum sesquichloride. Into that vessel 2 also an aqueous basic solution is introduced via line 3 so that the outlet stream and the aqueous basic solution may be mixed thoroughly to deactivate the catalytic components.

The catalyst components are transformed into corresponding non-reactive compounds according to the following reaction schemes:

ZrX₄+4NaOH → ZrO₂+4NaX+2H₂O

(C₂H₅)₃Al₂Cl₃+5NaOH+3H₂O → 3NaCl+3C₂H₆+2Na[Al(OH)₄]

The mixture obtained in the static mixing vessel is then transferred to separator 4, preferably a gravity separator, wherein phase separation takes place in that the aqueous basic phase (including deactivated dissolved catalytic components and precipitated ZrO₂) forms the lower phase, and the organic LAO-containing phase forming the upper phase. Aqueous and organic phase may be easily separated in a known manner. The aqueous basic phase may be recycled into the static mixing vessel 2 (preferably with prior removal of precipitated ZrO₂), or the aqueous basic solution may be further processed to selectively recover the aluminum compound (with prior removal of ZrO₂) by drying the aqueous basic solution in a drying section 5, or by neutralizing and precipitating in a neutralization and precipitation section (not shown). As an alternative, the aqueous basic solution containing the deactivated Al-compound may, after separation of the precipitated ZrO₂, be directly used as fine chemical. The organic phase obtained in the separator 4 may be transferred to a washing section 6 to clean the organic phase completely from any traces of basic solution or salts of deactivated catalytic components. The thus obtained organic phase comprising organic solvent, dissolved ethylene and linear alpha-olefins may be further processed as is well known by someone skilled in the art, e.g. by rectification.

In the above first scheme, X stands for any ligand providing an active zirconium catalyst for the oligomerisation of ethylene, preferably X is a carboxylate. The decomposed products are the corresponding salts of the catalyst components and are dissolved in the aqueous phase, except for zirconium which precipitates as solid ZrO₂.

The features disclosed in the foregoing description, in the claims or in the accompanying drawing may, both separately and in any combination thereof, be material for realizing the invention in diverse forms thereof.

## Claims

1. Method for deactivation and removal of catalytic components in the oligomerisation of ethylene to form linear alpha-olefins, comprising the steps of:
a) obtaining an organic phase outlet stream from an oligomerisation reactor comprising organic solvent, catalyst, dissolved ethylene and linear alpha-olefins;
b) mixing the outlet stream obtained in step a) with an aqueous phase basic solution to deactivate the catalyst compontents; and
c) separating the linear alpha-olefins containing organic phase from the aqueous basic phase, the aqueous basic phase containing the deactivated catalyst components.

2. Method according to claim 1, wherein mixing in step b) is carried out in a static mixer.

3. Method according to claim 1 or 2, wherein separating in step c) is carried out in a gravity separator.

4. Method according to any of the preceding claims, wherein the aqueous basic phase contains alkali metal hydroxide, preferably NaOH and/or KOH, NH₃, organic amines or mixtures thereof.

5. Method according to any of the preceding claims, wherein the aqueous basic phase obtained in step c) is recycled into mixing step b).

6. Method according to any of the preceding claims, wherein deactivated solid catalytic components are separated from deactivated dissolved catalytic components.

7. Method according to any of the preceding claims, wherein the solid catalytic components and/or the dissolved catalytic components obtained are dried, neutralized and precipitated.

8. Method according to any of the preceding claims, wherein the linear alpha-olefins containing organic phase obtained in step c) is washed with water.
